# EUROPÄISCHE PATENTSCHRIFT

(11) **EP 0 832 943 B1**
(45) Veröffentlichungstag und Bekanntmachung des Hinweises auf die Patenterteilung: **15.12.2004**
(21) Anmeldenummer: 97116595.6
(22) Anmeldetag: 24.09.1997
(51) Int. Cl.: C09C 1/00, C09C 3/12, C09D 7/12, C09D 11/00, C08K 9/06, C03C 4/02, C04B 33/14, A61K 7/00

(54) **Schwitzwasserbeständige blaustichige Glanzpigmente**
Brilliant bluish pigments resistant to condensed moisture
Pigments brillants bleuâtres résistant à l'humidité de condensation

(30) Priorität: 30.09.1996 DE 19640188
(43) Veröffentlichungstag der Anmeldung: 01.04.1998
(73) Patentinhaber: BASF AKTIENGESELLSCHAFT, 67056 Ludwigshafen (DE)
(72) Erfinder: Kaliba, Claus, Dr., 67141 Neuhofen (DE); Keller, Harald, Dr., 67069 Ludwigshafen (DE); Gonzalez Gomez, Juan Antonio, Dr., 67063 Ludwigshafen (DE); Bidlingmaier, Hermann, 77654 Offenburg (DE); Ellinghoven, Raymond, 71672 Marbach (DE); Schmid, Raimund, Dr., 67435 Neustadt-Mussbach (DE)

(56) Entgegenhaltungen:
- EP-A- 0 332 071
- EP-A- 0 498 458
- DE-A- 4 321 005
- DE-A- 19 511 697

## Beschreibung

Die vorliegende Erfindung betrifft neue, schwitzwasserbeständige blaustichige Glanzpigmente auf der Basis von in einer reduzierenden Atmosphäre erhitzten titandioxidbeschichteten Plättchen, welche durch Umsetzung der reduzierten Plättchen mit einem Silan der allgemeinen Formel I

RₐSiX_{b} I

in der die Variablen folgenden Bedeutung haben:
- R: C₁-C₄-Alkylreste, die in ω-Position durch eine Glycidoxygruppe, eine Aminogruppe oder eine Hydroxylgruppe oder eine Monoalkylaminogruppe oder einen Alkoxyrest, deren Alkylketten jeweils bis zu 4 Kohlenstoffatome enthalten können und durch ein Ethersauerstoffatom oder eine Iminogruppe unterbrochen sein können, substituiert sind, wobei die Reste R für a > 1 gleich oder verschieden sein können;
- X: C₁-C₄-Alkoxy;
- a: 1 oder 2;
- b: 2 oder 3, wobei die Summe a + b = 4 ist,
erhältlich sind.

Außerdem betrifft die Erfindung ein Verfahren zur Herstellung dieser Pigmente sowie ihre Verwendung zum Einfärben von Lacken, Druckfarben, Tinten, Kunststoffen, Gläsern, keramischen Produkten und Zubereitungen der dekorativen Kosmetik.

Reduzierte titandioxidbeschichtete Glimmerpigmente, deren TiO₂-Beschichtung reduzierte Titanspezies (Oxidationszahl des Titans < 4 bis 2) enthält oder gänzlich in diese reduzierten Spezies umgewandelt ist, sind schon länger als "dunkle" Perlglanzpigmente für den blauen bis schwarzen Farbtonbereich bekannt. Besonders blaustichige Glanzpigmente werden in der EP-A-332 071 und der nicht vorveröffentlichten DE-A-195 11 697 beschrieben. Diese Pigmente zeichnen sich durch gutes Deckvermögen, hohe Farbstärke und hohen Glanz aus und sind insbesondere für Automobillacke von Interesse. Oftmals weisen sie jedoch keine zufriedenstellende Schwitzwasserbeständigkeit auf.

Aus der DE-A-43 21 005 sind nichtreduzierte, TiO₂-beschichtete Glimmerpigmente bekannt, die für den Einsatz in wasserverdünnbaren Lacksystemen mit einer Deckschicht aus den Oxiden von Silicium, Aluminium und Cer und in einigen Fällen auch Zirkon und dem Hydrolyseprodukt eines Zirkonaluminats, Metallsäureesters oder organofunktionellen Silans als organischem Kupplungsreagenz versehen sind.

In der EP-A-498 458 werden ω-Aminoalkylalkoxysilane mit mindestens 6 Kohlenstoffatome enthaltenden Alkylketten beschrieben, die eingesetzt werden, um anorganische Materialien mit organischen Materialien verträglich zu machen.

Der Erfindung lag die Aufgabe zugrunde, schwitzwasserbeständige blaustichige Glanzpigmente auf Basis reduzierter titandioxidbeschichteter Silikatplättchen mit vorteilhaften Anwendungseigenschaften bereitzustellen.

Demgemäß wurden die eingangs definierten Glanzpigmente gefunden.

Außerdem wurde das hierdurch definierte Verfahren zur Herstellung dieser Glanzpigmente gefunden.

Weiterhin wurde die Verwendung dieser Glanzpigmente zur Einfärbung von Lacken, Druckfarben, Tinten, Kunststoffen, Gläsern, keramischen Produkten und Zubereitungen der dekorativen Kosmetik gefunden.

Für die erfindungsgemäßen Glanzpigmente als Substratmaterial dienende silikatische Plättchen sind insbesondere helle oder weiße Glimmer, wobei Schuppen von vorzugsweise naß vermahlenem Muskovit besonders bevorzugt sind. Selbstverständlich sind auch andere natürliche Glimmer, wie Phlogopit oder Biotit, künstliche Glimmer, Talk- und Glasschuppen geeignet.

Die silikatischen Plättchen sind mit einer im wesentlichen aus Titandioxid bestehenden Schicht belegt, die als untergeordnete Bestandteile (im allgemeinen < 5 Gew.-%) weitere, vorzugsweise farblose, Metalloxide wie Zirkondioxid, Zinndioxid, Aluminiumoxid und Siliciumdioxid enthalten kann.

Derartige Pigmente sind allgemein bekannt und unter dem Namen Iriodin® (E. Merck, Darmstadt), Flonac® (Kemira Oy, Pori, Finnland) oder Mearlin® (Mearl Corporation, Ossining, New York) im Handel.

Die Größe der Silikatplättchen ist an sich nicht kritisch und kann auf den jeweiligen Anwendungszweck abgestimmt werden. In der Regel haben die Plättchen mittlere größte Durchmesser von etwa 1 bis 200 µm, insbesonders etwa 5 bis 100 µm, und Dicken von etwa 0,1 bis 1 µm, insbesondere um etwa 0,5 µm. Ihre spezifische freie Oberfläche (BET) liegt üblicherweise bei 1 bis 15 m²/g, insbesondere 3 bis 12 m²/g.

Die Dicke der TiO₂-Schicht bestimmt die Reflexionsfarbe des Substratmaterials und beträgt vorzugsweise 50 bis 100 nm (silber) oder 300 bis 340 nm (blau; optische Schichtdicken).

Bei den erfindungsgemäßen Glanzpigmenten sind die als Substratmaterial dienenden titandioxidbeschichteten Silikatplättchen in einer reduzierenden Gasatmosphäre erhitzt worden.

Als reduzierende Gase eignen sich dabei z.B. Ammoniakgas, Wasserstoff, flüchtige Kohlenwasserstoffe (insbesondere C₁-C₄-Alkane) und deren Gemische. Alle Gase werden vorzugsweise im Gemisch mit Inertgasen wie Stickstoff eingesetzt (vgl. die nicht vorveröffentlichte DE-A-195 11 697 und die dort u.a. genannte EP-A-322 071).

Bevorzugte reduzierende Gase sind Ammoniakgas und Gemische von Ammoniakgas mit flüchtigen Kohlenwasserstoffen wie Methan, Ethan und/oder Propan, für die ein Volumenverhältnis von etwa 95:5 bis 70:30 zu empfehlen ist. Der Stickstoffanteil an der Gesamtgasmenge der jeweils besonders bevorzugten Reduktionsgas/Inertgas-Mischungen liegt vorzugsweise bei bis zu 90 Vol.-% bzw. bei 10 bis 60 Vol.-%.

Geeignete Reduktionstemperaturen betragen bei der Reduktion mit Ammoniakgas vorzugsweise 750 bis 850°C und bei der Umsetzung mit Ammoniakgas/Kohlenwasserstoff-Gemischen bevorzugt > 800 bis 900°C.

Bei der Reduktion werden reduzierte Titanspezies (niedere Titanoxide wie Ti₃O₅, Ti₂O₃ bis zu TiO, Titanoxynitride sowie Titannitrid) gebildet, die aufgrund ihrer blauen Absorptionsfarbe zusammen mit den silbern bzw. blau reflektierenden Substratplättchen besonders farbintensive, blaustichige Glanzpigmente ergeben.

Die reduzierten titandioxidbeschichteten Glimmerpigmente sind allgemein bekannt und auch im Handel unter dem Namen Paliocrom® (BASF, Ludwigshafen) erhältlich.

Die erfindungsgemäßen, schwitzwasserbeständigen blaustichigen Glanzpigmente können vorteilhaft nach dem erfindungsgemäßen Herstellungsverfahren durch Umsetzung der reduzierten titandioxidbeschichteten Silikatplättchen mit einem Silan der Formel I

RₐSiX_{b} I

erhalten werden.

Geeignete Reste R bedeuten dabei Alkylreste, die in der Regel 1 bis 4, bevorzugt 2 bis 4 Kohlenstoffatome enthalten und in ω-Position durch eine der folgenden Gruppen substituiert sind: eine Glycidoxygruppe, eine Aminogruppe, eine Hydroxylgruppe oder eine Monoalkylaminogruppe oder eine Alkoxygruppe, deren Alkylketten jeweils bis zu 4 Kohlenstoffatome enthalten können und die durch ein Ethersauerstoffatom oder eine Iminogruppe unterbrochen sein können.

Unter diesen Substituenten sind Mono- (C₁-C₄-alkyl)aminogruppen, deren Alkylrest durch ein Ethersauerstoffatom oder eine Iminogruppe substituiert sein kann, bevorzugt, die Glycidoxygruppe besonders bevorzugt und die Aminogruppe ganz besonders bevorzugt.

Enthält das Silan I mehrere Alkylreste R (a = 2), dann können diese gleich oder verschieden sein. Vorzugsweise ist jedoch nur ein Rest R enthalten (a = 1).

Die Reste X bedeuten gleiche Alkoxyreste, die in der Regel bis zu 4, bevorzugt bis zu 2 Kohlenstoffatome enthalten.

Geeignete Silane I enthalten mindestens 2 Alkoxyreste (b = 2), vorzugsweise jedoch 3 Alkoxyreste (b = 3).

Da die Umsetzung der reduzierten Silikatplättchen erfindungsgemäß bevorzugt mit in der Gasphase zersetztem Silan erfolgt, sind Silane der Formel I, die bei Temperaturen < 500°C, vorzugsweise < 400°C, einen ausreichend hohen Dampfdruck aufweisen und so eine einfache Verdampfung ermöglichen, besonders geeignet.

Besonders bevorzugte Silane entsprechen der Formel Ia

R'SiX'₃ Ia

in der R' einen Propylrest mit endständiger Aminogruppe oder endständiger Glycidoxygruppe und X' Methoxy oder Ethoxy bedeutet.

Beispiele für ganz besonders bevorzugte Silane sind 3-Aminopropyltriethoxysilan, 3-Aminopropyltrimethoxysilan und 3-Glycidoxypropyltrimethoxysilan.

Als Beispiele für weitere geeignete Silane seien N-(2-Aminoethyl)-3-aminopropyltrimethoxysilan, 2-Aminoethyltriethoxysilan, 4-Aminobutyltriethoxysilan und Di-(3-aminopropyl)diethoxysilan genannt.

Selbstverständlich können auch Mischungen der Silane I eingesetzt werden.

Bereits sehr geringe Mengen Silan I reichen aus, um die erfindungsgemäßen Pigmente schwitzwasserbeständig zu machen. Üblicherweise werden 0,5 bis 7 g, bevorzugt 2 bis 5 g, Silan je 100 g Pigment verwendet.

Die erfindungsgemäße Umsetzung der reduzierten Silikatplättchen mit dem Silan I kann naßchemisch bei Raumtemperatur in wäßriger Suspension erfolgen. Selbstverständlich kann man auch bei höheren Temperaturen von bis zu 95°C arbeiten, jedoch ist dies in der Regel nicht erforderlich.

Gewünschtenfalls kann der wäßrigen Suspension der Silikatplättchen noch ein nichtionisches Tensid zugesetzt werden, welches ebenfalls die Oberfläche der Silikatplättchen belegt und so zu besonders staubarmen Pigmenten führt. Bevorzugte Tenside sind hier z.B. Poly-C₂-C₃-alkylenglykole, insbesondere Polyethylenglykole mit einem mittleren Molekulargewicht von 200 bis 800 und Polypropylenglykole mit einem mittleren Molekulargewicht von 400 bis 1000, aber auch gemischte Polyethylen/Polypropylenglykole. Geeignete Tensidmengen betragen üblicherweise 2 bis 10 g je 100 g Pigment.

Verfahrenstechnisch geht man dabei zweckmäßigerweise so vor, daß man eine wäßrige Suspension der reduzierten Silikatplättchen in einem Reaktionsgefäß mit Rührwerk vorlegt und unter Rühren eine wäßrige Lösung des Silans, die gewünschtenfalls auch das Tensid enthalten kann, oder auch unverdünntes Silan zugibt.

Die Isolierung des mit dem Silan ungesetzten Pigments erfolgt vorteilhaft durch Sprühtrocknung. Es ist aber auch möglich, das Pigment abzufiltrieren und im Trockenschrank zu trocknen. Jedoch empfiehlt es sich hierbei, das restliche Wasser zunächst durch ein niedrigsiedendes, mit Wasser mischbares organisches Lösungsmittel auszutauschen, um ein Zusammenbacken der Pigmentplättchen beim Trocknen zu verhindern. Geeignete Lösungsmittel sind dabei z.B. Aceton und C₁-C₄-Alkohole wie Methanol und Ethanol.

Man kann die Silikatplättchen auch ohne Wasser in einen Feststoffmischer mit zusätzlichen Vorrichtungen zur Desagglomerierung geben und unter Rühren und Desagglomerieren eine wäßrige Lösung des Silans und gewünschtenfalls des Tensids auf die Pigmentplättchen aufsprühen.

Eine bevorzugte Ausführungsform des erfindungsgemäßen Verfahrens besteht in der Umsetzung der reduzierten Silikatplättchen mit gasförmigem (verdampftem) Silan (chemical vapor deposition, CVD).

Vorzugsweise wird auch die CVD-Variante in Gegenwart von Wasser (Wasserdampf) durchgeführt.

Als Reaktor kann für diese Verfahrensvariante vorteilhaft ein Wirbelschichtreaktor verwendet werden, in dem die reduzierten Silikatplättchen mit einem inerten Wirbelgas, bevorzugt Stickstoff, fluidisiert und gewünschtenfalls auf eine Temperatur von bis zu 300°C, vorzugsweise auf 150 bis 300°C, erhitzt werden. Das verdampfte Silan und Wasserdampf werden dann mit Hilfe inerter Trägergasströme (verschiedener Teilströme des Wirbelgases) aus vorgeschalteten Verdampfergefäßen über getrennte Düsen eingetragen.

Besonders geeignet für die CVD-Variante ist auch ein Feststoffmischer mit zusätzlichen Vorrichtungen zur Desagglomerierung, dem ebenfalls mit Inertgas durchspülte Verdampfer (z.B. Dünnschichtverdampfer) für Silan und Wasser vorgeschaltet sind. Als Beispiele für bevorzugte Feststoffmischer seien Mischer mit Pflugscharrührwerk und Vertikalwellenmischer, die jeweils mit Stator/Rotor-Schlagwerk ausgerüstet sind, genannt.

Die Verdampfertemperaturen hängen dabei natürlich vom Siedepunkt der zu verdampfenden Substanz ab. Weiterhin spielt auch die Stärke des durch den Verdampfer geleiteten Inertgasstromes sowie die gewünschte Silankonzentration im Reaktor eine Rolle. So betragen z.B. für 3-Aminopropyltriethoxysilan geeignete Verdampfertemperaturen etwa 70 bis 150°C bei Verwendung eines Wiriaelschichtreaktors bzw. etwa 150 bis 300°C bei der Umsetzung im Feststoffmischer (entsprechend Trägergasströmen von etwa 400 bis 200 l/h).

Bei Verwendung eines Wirbelschichtreaktors liegt die Konzentration des verdampften Silans zweckmäßigerweise bei ≤ 3 Vol.-%, vorzugsweise bei 0,001 bis 0,5 Vol.-%, jeweils bezogen auf die Gesamtgasmenge im Reaktor. Wird die Umsetzung in Gegenwart von Wasserdampf durchgeführt, dann sollte mindestens die stöchiometrisch zur Hydrolyse des Silans erforderlichen Menge Wasserdampf eingesetzt werden, bevorzugt ist jedoch ein 10 bis 100facher Überschuß.

Bei der Umsetzung in einem Feststoffmischer beträgt die Silankonzentration im Trägergasstrom in der Regel 0,1 bis 6 g/l, vorzugsweise 0,5 bis 4 g/l. Wird Wasserdampf zugesetzt, so ist hier ein 1 bis 10facher Überschuß bevorzugt. Dabei empfiehlt es sich, nach beendeter Silanverdampfung weiteren Wasserdampf zuzuführen, um eine vollständige Hydrolyse des eventuell adsorbierten Silans in das intermediär gebildete, reaktive Silanol zu gewährleisten.

Für die CVD-Verfahrensvariante übliche Reaktionszeiten betragen bei der Umsetzung in einem Feststoffmischer üblicherweise 2 bis 10 h, bei Verwendung eines Wirbelschichtreaktors kann die Reaktionszeit bis zu 20 h betragen.

Die erfindungsgemäßen blaustichigen Glanzpigmente zeichnen sich durch vorteilhafte Anwendungseigenschaften, insbesondere hohe Schwitzwasserbeständigkeiten, aus, wobei vor allem die in einem Feststoffmischer mit dem Silan umgesetzten Pigmente auch eine hervorragende Dispergierbarkeit in Lacken zeigen.

Sie eignen sich vorteilhaft für viele Zwecke, wie zur Einfärbung von Kunststoffen, Gläsern, keramischen Produkten, Zubereitungen der dekorativen Kosmetik, Tinten und Druckfarben und besonders von Lacken, insbesonders auch Automobillacken.

Für diese Anwendungszwecke lassen sich die erfindungsgemäßen Pigmente auch vorteilhaft in Abmischung mit transparenten und deckenden Weiß-, Bunt- und Schwarzpigmenten verwenden.

### Beispiele

### A) Herstellung von erfindungsgemäßen Glanzpigmenten

### Beispiel 1

In einem mit Pflugscharrührwerk, Stator/Rotor-Schlagwerk, 3 getrennten Gaszuleitungen und 2 vorgeschalteten Dünnschichtverdampfern versehenen Mischer der Fa. Lödige mit ca. 50 l Inhalt wurden 4 kg eines mit Ammoniakgas bei 800°C reduzierten silbern reflektierenden titandioxidbeschichteten Glimmerpigments in Gegenwart von Wasserdampf und Stickstoff mit 3-Aminopropyltriethoxysilan umgesetzt. Ein Stickstoffstrom von 300 l/h wurde über den auf 170°C erhitzten Silanverdampfer geleitet, ein Stickstoffstrom von 160 l/h wurde über den auf 85°C erhitzten Wasserverdampfer geführt. In 65 min wurden so 218 g Silan und 52 g Wasser zugeführt. Nach beendeter Silanverdampfung wurde noch weitere 80 min Wasserdampf eingeleitet, so daß insgesamt 117 g Wasser zugeführt wurden.

Das erhaltene Pigment hatte einen Kohlenstoffgehalt von 0,8 Gew.-%. Der bei Siebung < 50 µm anfallende Grobanteil betrug 2 Gew.-%.

### Beispiel 2

In dem Mischer aus Beispiel 1 wurden 4 kg des Glimmerpigments aus Beispiel 1 in Gegenwart von Wasserdampf und Stickstoff mit 3-Aminopropyltriethoxysilan umgesetzt. Mit einem Stickstoffstrom von jeweils 200 l/h wurden 120 g Silan aus dem auf 240°C erhitzten Verdampfer und 16 g Wasser aus dem auf 110°C erhitzten Verdampfer in 10 min zugeführt. Nach beendeter Silanverdampfung wurde noch weitere 50 min Wasserdampf eingeleitet, so daß insgesamt 95 g Wasser zugeführt wurden.

Das erhaltene Pigment hatte einen Kohlenstoffgehalt von 0,5 Gew.-%. Der bei Siebung < 50 µm anfallende Grobanteil betrug 0,7 Gew.-%.

### Beispiel 3

In einem Wirbelschichtreaktor aus Glas mit einem Durchmesser von 16 cm und einer Höhe von 100 cm mit Glasfrittenboden und oben eingehängten, mit einem Stickstoff-Jet abreinigenden Filterstrümpfen und zwei seitlich oberhalb des Frittenbodens eingebrachten Düsen zur Gaseinleitung wurden 600 g des Glimmerpigments aus Beispiel 1 unter Verwirbelung mit insgesamt 1700 l/h Stickstoff auf 200°C erhitzt. Dabei wurde ein Teil des Wirbelgases (400 l/h Stickstoff) durch eine auf 80°C erhitzte Verdampfervorlage mit 3-Aminopropyltriethoxysilan und ein weiterer Teil des Wirbelgases (300 l/h Stickstoff) durch eine auf 40°C temperierte Vorlage mit Wasser geleitet. Insgesamt wurden 35 g (37 ml) Silan zugeführt.

Das erhaltene Pigment hatte einen Kohlenstoffgehalt von 0,5 Gew.-%. Der bei Siebung < 50 µm anfallende Grobanteil betrug 0,5 Gew.-%.

### Beispiel 4

In dem Wirbelschichtreaktor aus Beispiel 3 wurden 600 g des Glimmerpigments aus Beispiel 1 unter Verwirbelung mit insgesamt 1800 l/h Stickstoff auf 200°C erhitzt. Dabei wurden 400 l/h Stickstoff durch eine auf 100°C erhitzte Verdampfervorlage mit 3-Aminopropyltriethoxysilan und 400 l/h durch eine auf 40°C temperierte Vorlage mit Wasser geführt. Insgesamt wurden 70 g (74 ml) Silan zugeführt.

Das erhaltene Pigment hatte einen Kohlenstoffgehalt von 1,1 Gew.-%. Der bei Siebung < 50 µm anfallende Grobanteil betrug 0,3 Gew.-%.

### Beispiel 5

In einem Behälter mit Rührwerk wurde zu einer Suspension von 100 kg des Glimmerpigments aus Beispiel 1 in 850 l Wasser eine Lösung von 5 kg 3-Aminopropyltriethoxysilan in 50 l Wasser zugegeben. Anschließend wurde das Wasser durch Sprühtrocknung entfernt. Dabei wurde die Suspension bei einem Druck von 4,5 bar, einer Turmeintrittstemperatur von 320°C und einer Turmaustrittstemperatur von 110°C mit einer Rate von 130 kg/h versprüht.

Das erhaltene Pigment ergab bei Siebung < 50 µm einen Grobanteil von 0,6 Gew.-%.

### Beispiel 6

Zu einer Suspension von 20 g des Glimmerpigments aus Beispiel 1 in 200 ml Wasser wurden unter Rühren 0,4 g 3-Aminopropyltrimethoxysilan gegeben. Anschließend wurde das Wasser durch Sprühtrocknung in einem Laboraggregat entfernt. Dabei wurde die Suspension bei einer Turmeintrittstemperatur von 230°C und einer Turmaustrittstemperatur von 95°C mit einer Rate von 0,5 kg/h versprüht.

Das erhaltene Pigment hatte einen Kohlenstoffgehalt von 0,3 Gew.-%. Der bei Siebung < 50 µm anfallende Grobanteil betrug 0,5 Gew.-%.

### Beispiel 7

Analog Beispiel 6 wurden 20 g des Glimmerpigments aus Beispiel 1 mit 0,4 g 3-Glycidoxypropyltrimethoxysilan umgesetzt.

Das erhaltene Pigment hatte einen Kohlenstoffgehalt von 0,5 Gew.-%.

### Beispiel 8

In einem mit Pflugscharrührwerk und Stator/Rotor-Schlagwerk versehenen Mischer der Fa. Lödige von 250 l Inhalt wurden 44,2 kg des Glimmerpigments aus Beispiel 1 unter Rühren und Desagglomerieren mit einer Mischung aus 3,3 kg 3-Aminopropyltriethoxysilan, 2,4 kg eines Polypropylenglykols mit dem mittleren Molekulargewicht 600 und 0,2 kg Wasser besprüht.

Das erhaltene Pigment hatte einen Kohlenstoffgehalt von 1,0 Gew.-%.

### B) Beurteilung von erfindungsgemäßen Glanzpigmenten

Zur Beurteilung der Schwitzwasserbestandigkeit der Pigmente im Lack wurden zunächst wie folgt Lackierungen hergestellt: Jeweils 4 g des erhaltenen Pigments wurden in 96 g eines Polyester-Mischlackes mit 21 Gew.-% Festkörpergehalt eingerührt und 15 min unter 1500 Upm mit einem Propellerrührer dispergiert. Danach wurde der Basislackansatz jeweils auf eine Spritzviskosität von 18 sec im DIN-Becher 4 (DIN 53 211) eingestellt und sowohl auf ein nicht grundiertes Aluminiumblech als auch auf ein Karosserieblech (Lackaufbau: Bonderblech (zinkphosphatiertes Stahlblech)/kathodische Tauchlackierung/Grundierung auf Polyester/Acetobutyratbasis) gespritzt. Nach einer kurzen Ablüftzeit wurde naß in naß mit einem Einkomponenten-Klarlack auf Basis Acrylat/Melaminharz (47 Gew.-% Festkörpergehalt, eingestellt auf 23 Sec-DIN 4) überlackiert. Nach 30-minütigem Ablüften bei Raumtemperatur wurde 30 min bei 130°C eingebrannt.

Die Beurteilung des Schwitzwasserverhaltens erfolgte anschließend nach dem Cleveland Humidity Test (Schwitzwassertest: DIN ISO 6270) und dem Wassereintauchtest (ISO 2812-2; Mai 1982).

Beim Schwitzwassertest wurden die lackierten Bleche in einem Cleveland Condensing Humidity Cabinet (Condensation Tester Q·C·T der Fa. The Q-Panel Company; Cleveland, Ohio, USA) plaziert. Das mit vollentsalztem Wasser gefüllte Bad wurde auf 70°C Wasserbadtemperatur eingestellt, und die Bleche wurden 24 h lang bei 100 % relativer Luftfeuchte kontinuierlich betaut.

Beim Wassereintauchtest wurden die lackierten Bleche nach 24-stündiger Lagerung bei Raumtemperatur 24 h in ein auf 80°C temperiertes Wasserbad gestellt.

Nach Abschluß der Belastung wurden die Bleche jeweils abgetrocknet und unverzüglich bezüglich der Farbänderung nach der Grauskala gemäß EN 20105-A02 beurteilt. Nach dieser Skala werden farblich unveränderte Lackierungen mit 5 und vollständig weiß angelaufene Lackierungen mit 1 bewertet.

Ebenso wurde die Glanzänderung nach der relativen Bewertungsskala gemäß ISO 4628/1 beurteilt. In dieser ebenfalls von 0 bis 5 reichenden Skala entspricht die Kennzahl 0 keinem Glanzverlust und die Kennzahl 5 sehr starkem Glanzverlust.

Die Ergebnisse dieser Untersuchungen sind in der folgenden Tabelle zusammengestellt, wobei zum Vergleich auch die Ergebnisse für das nicht mit dem Silan umgesetzte Ausgangspigment (V) aufgeführt sind.

**Tabelle**

| Pigment aus Beispiel | Cleveland Humidity Test | | Wassereintauchtest | |
|---|---|---|---|---|
| | Farbänderung | Glanzänderung | Farbänderung | Glanzänderung |
| 1 | 4 | 1 | 5 | 1 |
| 2 | 5 | 0 | 5 | 0 |
| 3 | 5 | 0 | 4-5 | 1 |
| 4 | 5 | 0 | 4-5 | 2 |
| 5 | 5 | 0 | 5 | 2 |
| 6 | 5 | 1 | 4 | 2 |
| 7 | 5 | 1 | 4-5 | 2 |
| 8 | 5 | 1 | 4 | 1 |
| V | 2 | 4-5 | 2 | 4 |

## Patentansprüche

1. Schwitzwasserbeständige blaustichige Glanzpigmente auf der Basis von in einer reduzierenden Atmosphäre erhitzten titandioxidbeschichteten silikatischen Plättchen, erhältlich durch Umsetzung der reduzierten Plättchen mit einem Silan der allgemeinen Formel I
RₐSiX_{b} I
in der die Variablen folgenden Bedeutung haben:
R C₁-C₄-Alkylreste, die in ω-Position durch eine Glycidoxygruppe, eine Aminogruppe oder eine Hydroxylgruppe oder eine Monoalkylaminogruppe oder einen Alkoxyrest, deren Alkylketten jeweils bis zu 4 Kohlenstoffatome enthalten können und durch ein Ethersauerstoffatom oder eine Iminogruppe unterbrochen sein können, substituiert sind, wobei die Reste R für a > 1 gleich oder verschieden sein können;
X C₁-C₄-Alkoxy;
a 1 oder 2;
b 2 oder 3, wobei die Summe a + b = 4 ist.

2. Glanzpigmente nach Anspruch 1, bei denen die reduzierten Plättchen mit einem Silan der allgemeinen Formel Ia
R'SiX'₃ Ia
in der
R' einen Propylrest, der endständig durch eine Glycidoxygruppe oder eine Aminogruppe substituiert ist, und
X' Methoxy oder Ethoxy bedeutet,
umgesetzt werden.

3. Glanzpigmente nach Anspruch 1 oder 2, bei denen die reduzierten Plättchen in Gegenwart von Wasser oder Wasserdampf mit dem Silan umgesetzt werden.

4. Glanzpigmente nach den Ansprüchen 1 bis 3, bei denen die reduzierten Plättchen mit verdampftem Silan umgesetzt werden.

5. Glanzpigmente nach den Ansprüchen 1 bis 4, bei denen die reduzierten Plättchen in einem Wirbelschichtreaktor mit verdampftem Silan umgesetzt werden.

6. Glanzpigmente nach den Ansprüchen 1 bis 4, bei denen die reduzierten Plättchen mit dem Silan in einem mit Vorrichtungen zur Desagglomerierung versehenen Feststoffmischer mit vorgeschalteten Verdampfern für das Silan und gewünschtenfalls für Wasser umgesetzt werden.

7. Glanzpigmente nach den Ansprüchen 1 bis 6, bei denen die titandioxidbeschichteten silikatischen Plättchen in einer reduzierten Atmosphäre, die Ammoniakgas, Wasserstoff oder flüchtige Kohlenwasserstoffe oder deren Gemische enthält, erhitzt worden sind.

8. Verfahren zur Herstellung von schwitzwasserbeständigen blaustichigen Glanzpigmenten gemäß Anspruch 1, **dadurch gekennzeichnet, daß** man die reduzierten Plättchen mit einem Silan der allgemeinen Formel I
RₐSiX_{b} I
in der die Variablen folgenden Bedeutung haben:
R C₁-C₄-Alkylreste, die in ω-Position durch eine Glycidoxygruppe, eine Aminogruppe oder eine Hydroxylgruppe oder eine Monoalkylaminogruppe oder einen Alkoxyrest, deren Alkylketten jeweils bis zu 4 Kohlenstoffatome enthalten können und durch ein Ethersauerstoffatom oder eine Iminogruppe unterbrochen sein können, substituiert sind, wobei die Reste R für a > 1 gleich oder verschieden sein können;
X C₁-C₄-Alkoxy;
a 1 oder 2;
b 2 oder 3, wobei die Summe a + b = 4 ist,
umsetzt.

9. Verwendung von schwitzwasserbeständigen blaustichigen Glanzpigmenten gemäß den Ansprüchen 1 bis 7 zur Einfärbung von Lacken, Druckfarben, Tinten, Kunststoffen, Gläsern, keramischen Produkten und Zubereitungen der dekorativen Kosmetik.

## Claims

1. Humidity-resistant bluish luster pigments based on titania-coated silicatic platelets heated in a reducing atmosphere, obtainable by reaction of the reduced platelets with a silane of the general formula I
RₐSiX_{b} I
where:
R is a C₁-C₄-alkyl radical substituted in the ω-position by a glycidoxy group, by an amino group, by a hydroxyl group or by a monoalkylamino group or an alkoxy radical whose alkyl chains may each contain up to 4 carbon atoms and may be interrupted by an ether oxygen atom or an imino group, the radicals R for a > 1 being identical or different;
X is C₁-C₄-alkoxy;
a is 1 or 2; and
b is 2 or 3, subject to the proviso that a + b = 4.

2. Luster pigments as claimed in claim 1, wherefor the reduced platelets are reacted with a silane of the general formula Ia
R'SiX'₃ Ia
where
R' is a propyl radical having a glycidoxy or an amino substituent in the terminal position, and
X' is methoxy or ethoxy.

3. Luster pigments as claimed in claim 1 or 2, wherefor the reduced platelets are reacted with the silane in the presence of water or water vapor.

4. Luster pigments as claimed in any of claims 1 to 3, wherefor the reduced platelets are reacted with vaporized silane.

5. Luster pigments as claimed in any of claims 1 to 4, wherefor the reduced platelets are reacted with vaporized silane in a fluidized bed reactor.

6. Luster pigments as claimed in any of claims 1 to 4, wherefor the reduced platelets are reacted with the silane in a solids mixer equipped with deagglomerating means and with upstream vaporizers for the silane and optionally water.

7. Luster pigments as claimed in any of claims 1 to 6, wherefor the titania-coated silicatic platelets have been heated in a reducing atmosphere comprising ammonia gas, hydrogen or volatile hydrocarbons or mixtures thereof.

8. A process for producing humidity-resistant bluish luster pigments as claimed in claim 1, **characterized in that** the reduced platelets are reacted with a silane of the general formula I
RₐSiX_{b} I
where:
R is a C₁-C₄-alkyl radical substituted in the ω-position by a glycidoxy group, by an amino group, by a hydroxyl group or by a monoalkylamino group or an alkoxy radical whose alkyl chains may each contain up to 4 carbon atoms and may be interrupted by an ether oxygen atom or an imino group, the radicals R for a > 1 being identical or different;
X is C₁-C₄-alkoxy;
a is 1 or 2; and
b is 2 or 3, subject to the proviso that a + b = 4.

9. The use of humidity-resistant bluish luster pigments as claimed in any of claims 1 to 7 for coloring paints, inks, including printing inks, plastics, glasses, ceramic products and decorative cosmetic preparations.

## Revendications

1. Pigments brillants bleutés résistants à l'eau de condensation, à base de paillettes silicatées revêtues de dioxyde de titane, que l'on a chauffées sous atmosphère réductrice et que l'on peut obtenir au moyen d'une réaction des paillettes réduites avec un silane de formule générale I
RₐSiX_{b} I
dans laquelle les variables prennent les significations suivantes :
R représente un groupe alkyle en C₁ à C₄ qui est substitué, en position ω, par un groupe glycidoxy, un groupe amino ou un groupe hydroxyle ou un groupe monoalkylamino ou un groupe alcoxy dont les chaînes alkyle peuvent posséder, à chaque fois, jusqu'à 4 atomes de carbone, et peuvent être interrompues par un atome d'oxygène d'une fonction éther ou par un groupe imino, où les groupes R avec a > 1 peuvent être identiques ou différents;
X représente un groupe alcoxy en C₁ à C₄;
a vaut 1 ou 2;
b vaut 2 ou 3, où la somme a + b = 4.

2. Pigments brillants selon la revendication 1, dans lesquels les paillettes réduites réagissent avec un silane de formule générale Ia
R'SiX'₃ Ia
dans laquelle
R' représente un groupe propyle qui est substitué, en position terminale, par un groupe glycidoxy ou un groupe amino, et
X' représente un groupe méthoxy ou éthoxy.

3. Pigments brillants selon la revendication 1 ou 2, dans lesquels les paillettes réduites réagissent avec le silane en présence d'eau ou de vapeur d'eau.

4. Pigments brillants selon les revendications 1 à 3, dans lesquels les paillettes réduites réagissent avec le silane évaporé.

5. Pigments brillants selon les revendications 1 à 4, dans lesquels les paillettes réduites réagissent avec le silane évaporé dans un réacteur à lit fluidisé.

6. Pigments brillants selon les revendications 1 à 4, dans lesquels les paillettes réduites réagissent avec le silane dans un mélangeur pour solides équipé de dispositifs destinés à une désagglomération, en employant, en amont, des évaporateurs pour le traitement du silane et, si souhaité, de l'eau.

7. Pigments brillants selon les revendications 1 à 6, dans lesquels les paillettes silicatées revêtues de dioxyde de titane sont chauffées sous atmosphère réductrice contenant de l'ammoniac, de l'hydrogène ou des hydrocarbures volatils, ou bien leurs mélanges.

8. Procédé pour la préparation de pigments brillants bleutés résistants à l'eau de condensation selon la revendication 1, **caractérisé en ce que** l'on fait réagir les paillettes réduites, avec un silane de formule générale I
RₐSiX_{b} I
dans laquelle les variables prennent les significations suivantes :
R représente un groupe alkyle en C₁ à C₄ qui est substitué, en position ω, par un groupe glycidoxy, un groupe amino ou un groupe hydroxyle ou un groupe monoalkylamino ou un groupe alcoxy dont les chaînes alkyle peuvent posséder, à chaque fois, jusqu'à 4 atomes de carbone, et peuvent être interrompues par un atome d'oxygène d'une fonction éther ou un groupe imino, où les groupes R avec a > 1 peuvent être identiques ou différents;
X représente un groupe alcoxy en C₁ à C₄;
a vaut 1 ou 2;
b vaut 2 ou 3, où la somme a + b = 4.

9. Mise en oeuvre des pigments brillants bleutés résistants à l'eau de condensation selon les revendications 1 à 7, pour la coloration de laques, d'encres d'impression, d'encres, de matières synthétiques, de verres, de produits céramiques et de préparations de cosmétique décoratif.
